# EUROPÄISCHE PATENTSCHRIFT

(11) **EP 2 524 685 B1**
(45) Veröffentlichungstag und Bekanntmachung des Hinweises auf die Patenterteilung: **29.03.2017**
(21) Anmeldenummer: 12165302.6
(22) Anmeldetag: 24.04.2012
(51) Int. Cl.: A61K 8/34, A61K 8/365, A61K 8/37, A61K 8/39, A61K 8/60, A61Q 5/06

(54) **MITTEL FÜR KERATINHALTIGE FASERN ENTHALTEND WASSER, MINDESTENS EINEN CARBONSÄUREESTER VON PENTAERYTHRITOL, MINDESTENS EIN DIOL UND MINDESTENS EIN POLYOL**
AGENT FOR FIBRES CONTAINING KERATIN THAT CONTAINS WATER, AT LEAST ONE CARBOXYLIC ACID ESTER OF PENTAERYTHRITOL, AT LEAST ONE DIOL AND AT LEAST ONE POLYOL
AGENT POUR FIBRES DE KÉRATINE CONTENANT DE L'EAU, AU MOINS UN ESTER D'ACIDE DE CARBONE DE PENTA-ÉRITHRITOL, AU MOINS UN DIOL ET AU MOINS UN POLYOL

(30) Priorität: 16.05.2011 DE 102011075863
(43) Veröffentlichungstag der Anmeldung: 21.11.2012
(73) Patentinhaber: Henkel AG & Co. KGaA, 40589 Düsseldorf (DE)
(72) Erfinder: KNAPPE, Thorsten, 22869 Schenefeld (DE); NOLL, Marcus, 22850 Norderstedt (DE)

(56) Entgegenhaltungen:
- WO-A1-01/17491
- WO-A1-98/24402
- JP-A- H05 306 212
- JP-A- H09 255 524
- US-A1- 2003 108 504
- DATABASE GNPD [Online] MINTEL; Januar 2009 (2009-01), "Conditioner", XP002735649, Database accession no. 1024191
- DATABASE GNPD [Online] MINTEL; Januar 2008 (2008-01), "Conditioner", XP002735650, Database accession no. 837329

## Beschreibung

Die vorliegende Erfindung betrifft Mittel zur temporären Umformung und zur gleichzeitigen Konditionierung keratinhaltiger Fasern, insbesondere menschlichem Haar.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Haarschäume, Fönwellen etc. erzielt werden.

Entsprechende Mittel zur temporären Formgebung enthalten als formgebende Komponente üblicherweise synthetische Polymere, sogenannte festigende Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Eine wichtige Eigenschaft eines Mittels zur temporären Verformung keratinischer Fasern, im Folgenden auch Stylingmittel genannt, besteht darin, den behandelten Fasern in der erzeugten Form einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge der eingesetzten fixierenden Verbindung (z.B. Wachse, Polymere) bestimmt, wobei jedoch auch ein Einfluss der weiteren Bestandteile des Stylingmittels gegeben sein kann. Idealerweise ergeben die fixierenden Verbindungen bei der Anwendung auf dem Haar einen Film, der einerseits der Frisur einen starken Halt verleiht, andererseits aber hinreichend flexibel ist, um bei Beanspruchung nicht zu brechen. Ist der Film zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt.

Stylingmittel zu entwickeln, die alle gewünschten Eigenschaften in Kombination aufweisen, bereitet nach wie vor Schwierigkeiten. Insbesondere gilt dies für die Kombination von ästhetischen Gesichtpunkten einerseits und starkem und flexiblem Halt andererseits. Um einen starken Halt zu vermitteln muss die fixierend wirkende Verbindung gut auf der keratinhaltigen Faser haften und einen hinreichend harten Film bilden. Dennoch darf der resultierende Film dem Faserkollektiv keine Taktilität eines Bretts verleihen, sondern soll den Fasern einen Grad an Flexibilität ermöglichen, ohne dass die aufgeprägte Formgebung des Faserkollektivs, d.h. z.B. eine Frisur, verlorengeht.

Aufgabe der vorliegenden Erfindung war es daher, ein Mittel zur temporären Verformung keratinischer Fasern zur Verfügung zu stellen, das sich durch einen hohen Haltegrad auszeichnet und Glanz sowie hervorragende Pflegeeigenschaften der keratinhaltigen Faser ermöglicht.

Die WO01/17491 A1 und die US2003/108504 A1 offenbaren Zusammensetzungen, welche Pentaerythritoltetraisostearat und Diol(e) enthalten.

In der JP H05 306212 A wird die Verwendung hoher Konzentrationen von Polyolen bei der Umformung von Haaren beschrieben.

Es wurde nunmehr überraschenderweise gefunden, dass dies durch die erfindungsgemäße Kombination von Inhaltsstoffen (*vide infra*) erreicht werden kann. Erfindungsgemäße Mittel lassen sich hervorragend auf dem Haar verteilen, verleihen einen perfekten Frisurenhalt, sowie natürlich glänzende und gleichmäßig gepflegte Keratinfasern.

Ein erster Gegenstand der vorliegenden Erfindung sind daher Mittel zur Behandlung keratinhaltiger Fasern, insbesondere menschlichem Haar, enthaltend in Mengen, jeweils bezogen auf das Gewicht des gesamten Mittels, von
(a) 0,01 bis 10,0 Gew.-% mindestens eines Carbonsäureesters der Formel (I) 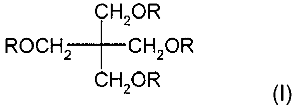 worin R unabhängig voneinander für eine verzweigte (C₈ bis C₃₀)-Acylgruppe oder ein Wasserstoffatom steht und höchstens einer der Reste R für ein Wasserstoffatom steht,
(b) 0,1 bis 30 Gew.-% mindestens eines (C₂ bis C₆)-Diols,
(c) 5,0 bis 20,0 Gew.-% mindestens eines Polyols,
(d) Wasser,
wobei zusätzlich mindestens ein anionisches Tensid in einer Menge von 1,0 bis 15,0 Gew.-% enthalten ist, wobei das anionische Tensid ein Alkenyletherphosphat der Formel (T1),

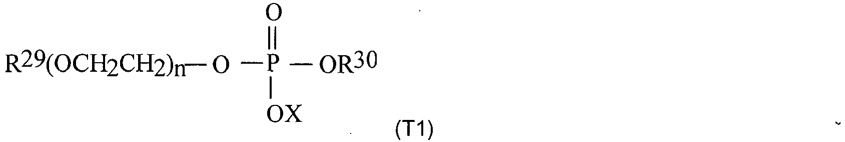

in der R²⁹ bevorzugt für einen ungesättigten Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄-Kohlenwasserstoffrest, steht, ist.

Soweit nicht anders definiert, beziehen sich alle Mengenangaben in Gewichtsprozent (Gew.-%) auf das Gewicht des gesamten Mittels.

Bevorzugter Weise stehen alle Reste R der Formel (I) unabhängig voneinander für eine verzweigte (C₈ bis C₃₀)-Acylgruppe. Es ist besonders bevorzugt, wenn gemäß Formel (I) R unabhängig voneinander steht für 2-Ethylhexanoyl, 2-Octyldodecanoyl, Isostearoyl, Isocetanoyl, Cetostearoyl, Hexyllauroyl oder Isononanoyl, insbesondere für Isostearoyl. Erfindungsgemäß besonders ausgeprägte Effekte zeigten sich, wenn der Carbonsäureester (a) ausgewählt wird, aus mindestens einer Verbindung der Formel (I), in der alle Reste R für eine verzweigte (C₈ bis C₃₀)-Acylgruppe, insbesondere die zuvor genannten bevorzugten Reste R, stehen.
Ein ganz besonders bevorzugt geeigneter Carbonsäureester (a) ist Pentaerythrityltetraisostearat (INCI-Bezeichnung Pentaerythrityl Tetraisostearate). Dieser Carbonsäureester wird beispielsweise unter dem Handelsnamen Crodamol^{®} PTIS von der Firma Croda vertrieben. Es ist erfindungsgemäß besonders bevorzugt, wenn die Mittel die Carbonsäureester der Formel (I) bzw. deren bevorzugte Vertreter in einer Menge von 0,1 bis 1,0 Gew.-% enthalten.

Bevorzugte Mittel sind außerdem dadurch gekennzeichnet, dass die (C₂ bis C₆)-Diole ausgewählt werden aus mindestens einem Diol der Gruppe, die gebildet wird aus 1,2-Propylenglykol, Butan-1,3-diol, Butan-2,3-diol, 2-Methyl-2,4-butandiol, 2-Methyl-2,4-pentandiol, 1,5-Pentandiol, 2-Ethyl-1,3-hexandiol. Dabei sind 1,2-Propylenglykol, Butan-1,3-diol oder deren Mischung besonders bevorzugt. Besonders bevorzugte Mittel enthalten die (C₂ bis C₆)-Diole in einer Menge von 2,0 bis 10,0 Gew.-%.

Weiterhin enthält das erfindungsgemäße Mittel zwingend mindestens ein Polyol. Ein Polyol enthält erfindungsgemäß mindestens drei Hydroxylgruppen. Es ist erfindungsgemäß bevorzugt, wenn zumindest ein in dem erfindungsgemäßen Mittel enthaltenes Polyol bei 25°C und einem Druck von 1 atm ein Feststoff ist.

Bevorzugte Polyole weisen ferner eine chemische Struktur auf, die ausschließlich aus Kohlenstoffatomen, Wasserstoffatomen und Sauerstoffatomen aufgebaut ist und ein Kohlenwasserstoffgerüst aufweist, an das mindestens drei Hydroxygruppen (insbesondere drei bis sechs Hydroxygruppen) binden. Dabei binden wiederum bevorzugt die Sauerstoffatome lediglich als Hydroxygruppen (-OH) und/oder als Etherfunktionalitäten (-O-) an das Kohlenwasserstoffgerüst.

Gemäß einer erfindungsgemäß bevorzugten Ausführungsform weisen die Moleküle der Polyole (c) pro Kohlenstoffatom des Kohlenwasserstoffgerüsts mindestens 0.8 Hydroxygruppen, besonders bevorzugt mindestens 0.9 Hydroxygruppe, ganz besonders bevorzugt 1 Hydroxygruppe auf.
Ferner ist es erfindungsgemäß bevorzugt, wenn alle im Molekül des linearen, apliphatischen Polyols vorhandenen Sauerstoffatome in Form von Hydroxygruppen vorliegen.
Außerdem erwiesen sich im Rahmen der Erfindung solche Polyole (c) als bevorzugt geeignet, deren molares Kohlenstoff zu Sauerstoffverhältnis im Bereich von 0.8 bis 1.5, insbesondere im Bereich von 0.9 bis 1.2, ganz bevorzugt bei 1 liegt.
Allerdings hat es sich als bevorzugt herausgestellt, dass, wenn das molare Verhältnis von Kohlenstoffatomen zu Sauerstoffatomen im Molekül des Polyols (c) 1 beträgt, das molare Verhältnis von Wasserstoffatomen zu Sauerstoffatomen im Molekül des Polyols (c) größer als 2 ist. Es ist erfindungsgemäß bevorzugt, wenn das Polyol (c) ausgewählt wird aus der Gruppe, die gebildet wird aus Glyzerin, 1,2,4-Pentantriol, 1,3,4-Pentantriol, 1,3,5-Pentantriol, Sorbitol, Inositol, Xylitol, Mannitol, Gluconolacton, Glucuronsäure, 1,2,6-Hexantriol, Hydroxyethylsorbitol, Phytantriol, Hydroxypropylphytantriol, Lactitol, Maltitol, Pentaerythritol, Polyglycerin-3, Glucose, Lactose, Maltose, Polyglycerin-4, Polyglycerin-6, Polyglycerin-10, Polyglycerin-20, Polyglycerin-40, Tripropylenglykol. Ein ganz besonders bevorzugtes Polyol (c) ist Sorbitol und/oder Glyzerin, wobei eine Mischung aus Sorbitol und Glyzerin wiederum als Polyol (c) bevorzugt wird.

Die erfindungsgemäßen Mittel enthalten bevorzugt mindestens 30 Gew.-%, insbesondere mindestens 40 Gew.-% Wasser.

Das erfindungsgemäße Mittel enthält in einer weiteren Ausführungsform zusätzlich mindestens ein filmbildendes und/oder festigendes Polymer.
Zu den bevorzugten Eigenschaften der filmbildenden Polymeren zählt die Filmbildung. Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Makeup, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.
Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.
Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger,

Haarschäume, Haarwachse, Haarsprays. Die Filmbildung kann dabei durchaus punktuell sein und nur einige Fasern miteinander verbinden.
Als eine Testmethode für die festigende Wirkung eines Polymers wird häufig der so genannte curlretention - Test angewendet.
Da Polymere häufig multifunktional sind, das heißt mehrere anwendungstechnisch erwünschte Wirkungen zeigen, finden sich zahlreiche Polymere in mehreren auf die Wirkungsweise eingeteilten Gruppen, so auch im CTFA Handbuch.
Das erfindungsgemäße Mittel enthält bevorzugt mindestens ein filmbildendes und/oder festigendes Polymer, das aus mindestens einem Polymer der Gruppe ausgewählt wird, die gebildet wird aus nichtionischen Polymeren, kationischen Polymeren, amphoteren Polymeren, zwitterionischen Polymeren und anionischen Polymeren.

Die zusätzlichen filmbildenden und/oder festigenden Polymere sind in dem erfindungsgemäßen Mittel bevorzugt in einer Menge von 0,01 Gew.-% bis 20 Gew.-%, insbesondere von 0,5 Gew.-% bis 15 Gew.-%, ganz besonders bevorzugt von 2,0 Gew.-% bis 10,0 Gew.-%, jeweils bezogen auf das Gewicht des Mittels, enthalten. Diese Mengenangaben gelten auch für alle in den erfindungsgemäßen Mitteln einsetzbaren nachfolgenden bevorzugten Typen der filmbildenden und/oder festigenden Polymere. Falls nachfolgend abweichende bevorzugte Mengen spezifiziert wurden, gelten letztere als wiederum noch bevorzugtere Mengen.

Besonders bevorzugt eignen sich erfindungsgemäß solche Mittel, die neben den zuvor definierten amphiphilen, kationischen Polymeren zusätzlich mindestens ein filmbildendes und/oder festigende Polymer enthalten, welches aus mindestens einem Polymer der Gruppe ausgewählt wird, die gebildet wird aus
- nichtionischen Polymeren auf Basis ethylenisch ungesättigter Monomere, insbesondere aus
   - Homopolymeren des N-Vinylpyrrolidons,
   - nichtionischen Copolymeren des N-Vinylpyrrolidons,
   - Homopolymeren und nichtionischen Copolymeren des N-Vinylcaprolactams,
   - Copolymeren des (Meth)acrylamids,
   - Polyvinylalkohol, Polyvinylacetat,
- Chitosan und Derivaten des Chitosans,
- kationischen Cellulosederivaten,
- kationischen Copolymeren des 3-(C₁ bis C₆)-Alkyl-1-vinyl-imidazoliniums,
- Homopolymeren und Copolymeren enthaltend die Struktureinheit der Formel (M-1) 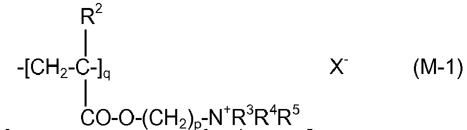 in der R²= -H oder -CH₃ ist, R³, R⁴ und R⁵ unabhängig voneinander ausgewählt sind aus (C₁ bis C₄)-Alkyl-, (C₁ bis C₄)-Alkenyl- oder (C₂ bis C₄)-Hydroxyalkylgruppen, p = 1, 2, 3 oder 4, q eine natürliche Zahl und X⁻ ein physiologisch verträgliches organisches oder anorganisches Anion ist,
- anionischen Polymeren, welche Carboxylat- und/oder Sulfonatgruppen aufweisen,
- anionischen Polyurethanen.

Bevorzugt als zusätzliches filmbildendes und/oder festigendes Polymer geeignete nichtionische Polymere auf Basis ethylenisch ungesättigter Monomere, sind solche nichtionischen Polymere, welche mindestens eine der nachfolgenden Struktureinheiten enthalten 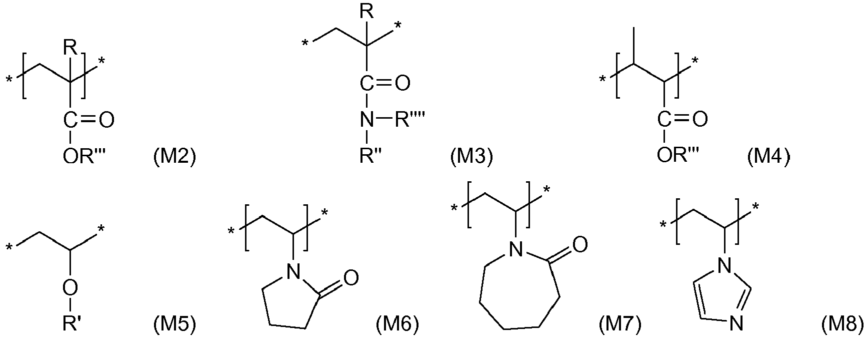 worin
- R: steht für ein Wasserstoffatom oder eine Methylgruppe,
- R': steht für ein Wasserstoffatom oder eine (C₁ bis C₄)-Acylgruppe,
- R" und R"": stehen unabhängig voneinander für eine (C₁ bis C₇)-Alkylgruppe oder ein Wasserstoffatom
- R'": steht für eine lineare oder verzweigte (C₁ bis C₄)-Alkylgruppe oder eine (C₂ bis C₄)-Hydroxyalkylgruppe.

Bevorzugte, nichtionische filmbildende und/oder nichtionische haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl-und Dialkylacrylamid, Alkyl-und Dialkylmethacrylamid, Alkylacrylat, Alkylmethacrylat, wobei jeweils die Alkylgruppen dieser Monomere aus (C₁ bis C₃)-Alkylgruppen ausgewählt werden.

Für die erfindungsgemäßen Mittel besonders geeignete nichtionische Polymere auf Basis ethylenisch ungesättigter Monomere enthalten mindestens eine der nachfolgenden Struktureinheiten 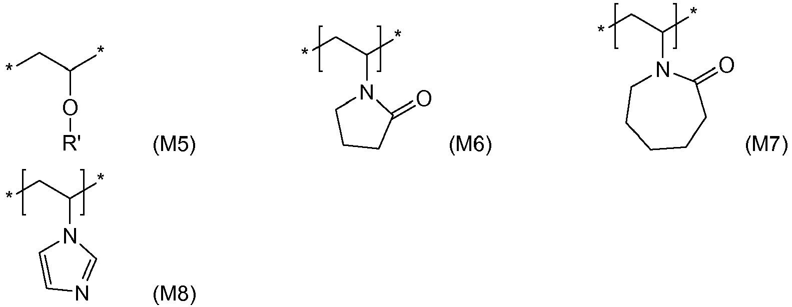 worin
- R': steht für ein Wasserstoffatom oder eine (C₁- bis C₃₀)-Acylgruppe, insbesondere für ein Wasserstoffatom oder eine Acetylgruppe.

Geeignet sind insbesondere Homopolymere des Vinylcaprolactams oder des Vinylpyrrolidons (wie beispielsweise Luviskol^{®} K 90 oder Luviskol^{®} K 85 der Firma BASF SE), Copolymerisate aus Vinylpyrrolidon und Vinylacetat (wie sie beispielsweise unter dem Warenzeichen Luviskol^{®} VA 37, Luviskol^{®} VA 55, Luviskol^{®} VA 64 und Luviskol^{®} VA 73 von der Firme BASF SE vertrieben werden), Terpolymere aus Vinylpyrrolidon, Vinylacetat und Vinylpropionat, Polyacrylamide (wie beispielsweise Akypomine^{®} P 191 von der Firma CHEM-Y), Polyvinylalkohole (die beispielsweise unter den Handelsbezeichnungen Elvanol^{®} von Du Pont oder Vinol^{®} 523/540 von der Firma Air Products vertrieben werden), Terpolymere aus Vinylpyrrolidon, Methacrylamid und Vinylimidazol (wie beispielsweise Luviset^{®} Clear der Firma BASF SE).
Neben den auf ethylenisch ungesättigten Monomeren basierenden nichtionischen Polymeren eignen sich weiterhin zur bevorzugten Ausführung der technischen Lehre nichtionische Cellulosederivate als filmbildende und/oder festigende Polymere, die bevorzugt ausgewählt werden aus Methylcellulose und insbesondere aus Celluloseether, wie Hydroxypropylcellulose (z. B. Hydroxypropylcellulose mit einem Molekulargewicht von 30.000 bis 50.000 g/mol, welche beispielsweise unter der Handelsbezeichnung Nisso SI^{®} von der Firma Lehmann & Voss, Hamburg, vertrieben wird), Hydroxyethylcellulose, wie sie beispielsweise unter den Warenzeichen Culminal^{®} und Benecel^{®} (AQUALON) und Natrosol^{®}-Typen (Hercules) vertrieben werden.

Besonders bevorzugte erfindungsgemäße Mittel sind dadurch gekennzeichnet, dass als filmbildendes und/oder festigendes Polymer ein nichtionisches Polymer enthalten ist, ausgewählt aus Homopolymeren des N-Vinylpyrrolidons und/oder aus Copolymeren des N-Vinylpyrrolidons. Mittel, die als filmbildendes nichtionisches und/oder festigendes nichtionisches Polymer mindestens ein Polymer ausgewählt aus der Gruppe, die gebildet wird aus
- Polyvinylpyrrolidon (am bevorzugtesten),
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymere aus N-Vinylpyrrolidon und N-Vinylimidazol und Methacrylamid,
- Copolymere aus N-Vinylpyrrolidon und N-Vinylimidazol und Acrylamid,
- Copolymere aus N-Vinylpyrrolidon mit N,N-Di(C₁ bis C₄)-Alkylamino-(C₂ bis C₄)-alkylacrylamid, enthalten, sind erfindungsgemäß ganz besonders bevorzugt.

Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Ein erfindungsgemäß bevorzugt geeignetes kationisches filmbildendes und/oder kationisches festigendes Polymer ist mindestens ein kationisches filmbildendes und/oder kationisches festigendes Polymer, das mindestens ein Strukturelement der Formel (M9) und zusätzlich mindestens ein Strukturelement der Formel (M10) enthält 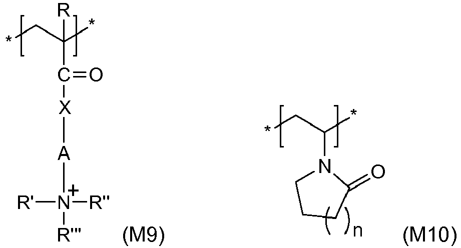 worin
- R: für ein Wasserstoffatom oder eine Methylgruppe steht,
- R', R" und R": unabhängig voneinander stehen für eine (C₁ bis C₃₀)-Alkylgruppe,
- X: steht für ein Sauerstoffatom oder eine Gruppe NH,
- A: steht für eine Ethan-1,2,-diylgruppe oder eine Propan-1,3-diylgruppe,
- n: 1 oder 3 bedeutet.

Solche Verbindungen sind beispielsweise als Copolymere aus mit Diethylsulfat quaterniertem Dimethylaminoethylmethacrylat, mit Vinylpyrrolidon mit der INCI-Bezeichnung Polyquaternium-11 unter den Bezeichnungen Gafquat^{®} 440, Gafquat^{®}734, Gafquat^{®}755 (jeweils Firma ISP) sowie Luviquat PQ 11 PN (Firma BASF SE) im Handel erhältlich.

Weiterhin werden die kationischen filmbildenden und/oder kationischen festigenden Polymere erfindungsgemäß besonders bevorzugt aus kationischen, quaternisierten Cellulose-Derivaten ausgewählt. Ferner eignen sich als filmbildendes und/oder festigendes Polymere bevorzugt kationische, quaternisierte Cellulosederivate. Es erweisen sich solche kationischen, quaternisierten Cellulosen als im Sinne der Erfindung besonders vorteilhaft, die in einer Seitenkette mehr als eine permanente kationische Ladung tragen. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat^{®} H 100, Celquat^{®} L 200 von der Firma National Starch vertrieben werden.

Als im Sinne der Erfindung besonders bevorzugt verwendbare kationische Polymere dienen weiterhin solche kationischen filmbildenden und/oder kationischen festigenden Copolymere, die mindestens ein Strukturelement der Formel (M11) 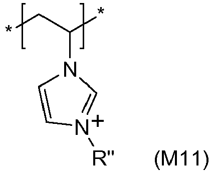 worin
R für eine (C₁ bis C₄)-Alkylgruppe, insbesondere eine Methylgruppe, steht, und zusätzlich mindestens ein weiteres kationisches und/oder nichtionisches Strukturelement aufweisen.
Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Es ist wiederum erfindungsgemäß bevorzugt, wenn als zusätzliches kationisches filmbildendes und/oder kationisches festigendes Polymer, mindestens ein Copolymer (e1) enthalten ist, das neben mindestens einem Strukturelement der Formel (M11) zusätzlich ein Strukturelement der Formel (M6) umfasst 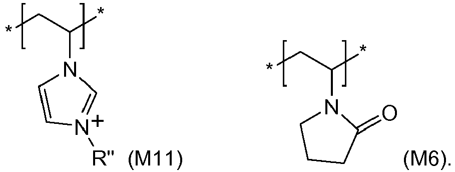 worin
R für eine (C₁ bis C₄)-Alkylgruppe, insbesondere eine Methylgruppe, steht.
Ganz besonders bevorzugte kationische filmbildende und/oder kationische festigende Polymere als Copolymere (e1) enthalten 10 bis 30 Mol-%, vorzugsweise 15 bis 25 Mol.-% und insbesondere 20 Mol.-% Struktureinheiten gemäß Formel (M11) und 70 bis 90 Mol.-%, vorzugsweise 75 bis 85 Mol.-% und insbesondere 80 Mol.-% Struktureinheiten gemäß Formel (M6).

Hierbei ist besonders bevorzugt, wenn die Copolymere (e1) neben Polymereinheiten, die aus dem Einbau der genannten Struktureinheiten gemäß Formel (M11) und (M6) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (e1) ausschließlich aus Struktureinheiten der Formel (M11) mit R" = Methyl und (M6) aufgebaut und lassen sich durch die allgemeine Formel (Poly1) 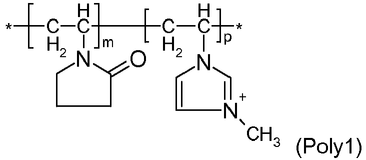 beschreiben, wobei die Indices m und p je nach Molmasse des Polymers variieren und nicht bedeuten sollen, daß es sich um Blockcopolymere handelt. Vielmehr können Struktureinheiten der Formel (M11) und der Formel (M6) im Molekül statistisch verteilt vorliegen.

Wird in zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat^{®} Style, Luviquat^{®} FC 370, Luviquat^{®} FC 550, Luviquat^{®} FC 905 und Luviquat^{®} HM 552. Wird in zur Kompensation der positiven Ladung des Polymers der Formel (Poly1) ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der BASF unter den Handelsnamen Luviquat^{®} UltraCare erhältlich.

Besonders bevorzugte erfindungsgemäße Mittel enthalten ein Copolymer (e1), insbesondere der Formel (Poly1), das Molmassen innerhalb eines bestimmten Bereiches aufweist. Hier sind erfindungsgemäße Mittel bevorzugt, bei denen das Copolymer (e1) eine Molmasse von 50 bis 400 kDa, vorzugsweise von 100 bis 300 kDa, weiter bevorzugt von 150 bis 250 kDa und insbesondere von 190 bis 210 kDa aufweist.

Zusätzlich zu dem bzw. den Copolymer(en) (e1) oder an dessen bzw. deren Stelle können die erfindungsgemäßen Mittel auch Copolymere (e2) enthalten, die ausgehend vom Copolymer (e1) als zusätzliche Struktureinheiten Struktureinheiten der Formel (M7) aufweisen

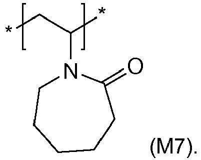

Weitere besonders bevorzugte erfindungsgemäße Mittel sind somit dadurch gekennzeichnet, daß sie als kationisches filmbildendes und/oder kationisches festigendes Polymer mindestens ein Copolymer (e2) enthalten, das mindestens eine Struktureinheit gemäß Formel (M11-a) und mindestens eine Struktureinheit gemäß Formel (M6) und mindestens weitere Struktureinheit gemäß Formel (M7) enthält

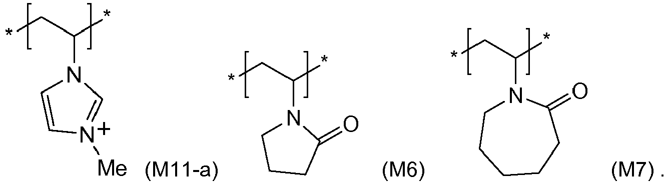

Auch hierbei ist besonders bevorzugt, wenn die Copolymere (e2) neben Polymereinheiten, die aus dem Einbau der genannten Struktureinheiten gemäß Formel (M11-a), (M6) und (M7) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (e2) ausschließlich aus Struktureinheiten der Formeln (M11-a), (M6) und (M7) aufgebaut und lassen sich durch die allgemeine Formel (Poly2) 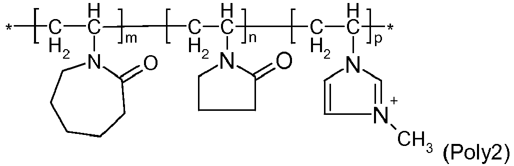 beschreiben, wobei die Indices m, n und p je nach Molmasse des Polymers variieren und nicht bedeuten sollen, daß es sich um Blockcopolymere handelt. Vielmehr können Struktureinheiten der besagten Formeln im Molekül statistisch verteilt vorliegen. Wird in zur Kompensation der positiven Ladung des Polymer der Formel (Poly2) ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat^{®} Hold erhältlich.

Ganz besonders bevorzugte Copolymere (e2) enthalten 1 bis 20 Mol-%, vorzugsweise 5 bis 15 Mol.-% und insbesondere 10 Mol.-% Struktureinheiten gemäß Formel (M11-a) und 30 bis 50 Mol.-%, vorzugsweise 35 bis 45 Mol.-% und insbesondere 40 Mol.-% Struktureinheiten gemäß Formel (M6) und 40 bis 60 Mol.-%, vorzugsweise 45 bis 55 Mol.-% und insbesondere 60 Mol.-% Struktureinheiten gemäß Formel (M7). Besonders bevorzugte erfindungsgemäße Mittel enthalten ein Copolymer (e2), das Molmassen innerhalb eines bestimmten Bereiches aufweist. Hier sind erfindungsgemäße Mittel bevorzugt, bei denen das Copolymer (e2) eine Molmasse von 100 bis 1000 kDa, vorzugsweise von 250 bis 900 kDa, weiter bevorzugt von 500 bis 850 kDa und insbesondere von 650 bis 710 kDa aufweist.

Zusätzlich zu dem bzw. den Copolymer(en) (e1) und/oder (e2) oder an dessen bzw. deren Stelle können die erfindungsgemäßen Mittel als filmbildendes kationische und/oder festigendes kationisches Polymer auch Copolymere (e3) enthalten, die als Struktureinheiten Struktureinheiten der Formeln (M11-a) und (M6) aufweisen, sowie weitere Struktureinhieten aus der Gruppe der Vinylimidazol-Einheiten und weitere Struktureinheiten aus der Gruppe der Acrylamid- und/oder Methacrylamid-Einheiten.

Weitere besonders bevorzugte erfindungsgemäße Mittel sind dadurch gekennzeichnet, daß sie als zusätzliches kationisches filmbildendes und/oder kationisches festigendes Polymer mindestens ein Copolymer (e3) enthalten, das mindestens eine Struktureinheit gemäß Formel (M11-a) und mindestens eine Struktureinheit gemäß Formel (M6) und mindestens eine Struktureinheit gemäß Formel (M10) und mindestens eine Struktureinheit gemäß Formel (M12) enthält

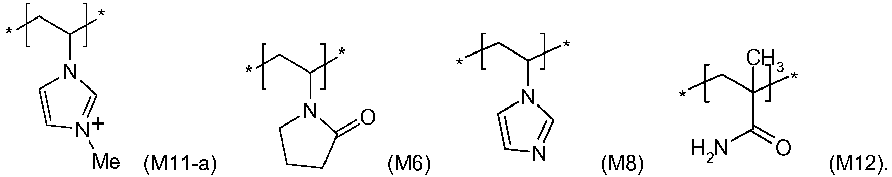

Auch hierbei ist besonders bevorzugt, wenn die Copolymere (e3) neben Polymereinheiten, die aus dem Einbau der genannten Struktureinheiten gemäß Formel (M11-a), (M6), (M8) und (M12) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (e3) ausschließlich aus Struktureinheiten der Formel (M11-a), (M6), (M8) und (M12) aufgebaut und lassen sich durch die allgemeine Formel (Poly3) 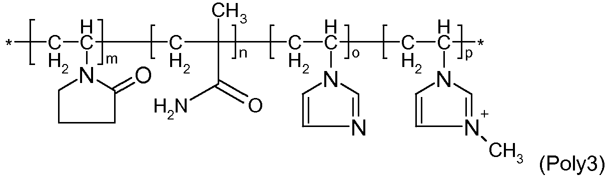 beschreiben, wobei die Indices m, n, o und p je nach Molmasse des Polymers variieren und nicht bedeuten sollen, daß es sich um Blockcopolymere handelt. Vielmehr können Struktureinheiten der Formeln (M11-a), (M6), (M8) und (M12) im Molekül statistisch verteilt vorliegen. Wird in zur Kompensation der positiven Ladung des Polymer der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat^{®} Supreme erhältlich.

Ganz besonders bevorzugte Copolymere (e3) enthalten 1 bis 12 Mol-%, vorzugsweise 3 bis 9 Mol.-% und insbesondere 6 Mol.-% Struktureinheiten gemäß Formel (M11-a) und 45 bis 65 Mol.-%, vorzugsweise 50 bis 60 Mol.-% und insbesondere 55 Mol.-% Struktureinheiten gemäß Formel (M6) und 1 bis 20 Mol.-%, vorzugsweise 5 bis 15 Mol.-% und insbesondere 10 Mol.-% Struktureinheiten gemäß Formel (M8) und 20 bis 40 Mol.-%, vorzugsweise 25 bis 35 Mol.-% und insbesondere 29 Mol.-% Struktureinheiten gemäß Formel (M12). Besonders bevorzugte erfindungsgemäße Mittel enthalten ein Copolymer (e3), das Molmassen innerhalb eines bestimmten Bereiches aufweist. Hier sind erfindungsgemäße Mittel bevorzugt, bei denen das Copolymer (e3) eine Molmasse von 100 bis 500 kDa, vorzugsweise von 150 bis 400 kDa, weiter bevorzugt von 250 bis 350 kDa und insbesondere von 290 bis 310 kDa aufweist.

Unter den zusätzlichen filmbildenden kationischen und/oder festigenden Polymer ausgewählt aus den kationischen Polymeren mit mindesten einem Strukturelement der obigen Formel (M11-a), gelten als bevorzugt: Vinylpyrrolidon/1-Vinyl-3-methyl-1H-imidazoliumchlorid-Copolymere (wie beispielsweise das mit der INCI-Bezeichnung Polyquaternium-16 unter den Handelsbezeichnungen Luviquat^{®} Style, Luviquat^{®} FC 370, Luviquat^{®} FC 550, Luviquat^{®} FC 905 und Luviquat^{®} HM 552 (BASF SE)), Vinylpyrrolidon/1-Vinyl-3-methyl-1H-imidazoliummethylsulfat-Copolymere (wie beispielsweise das mit der INCI-Bezeichnung Polyquaternium-44 unter den Handelsbezeichnungen Luviquat^{®} Care (BASF SE)), Vinylpyrrolidon/Vinylcaprolactam/1-Vinyl-3-methyl-1H-imidazolium-Terpolymer (wie beispielsweise das mit der INCI-Bezeichnung Polyquaternium-46 unter den Handelsbezeichnungen Luviquat^{®} Care oder Luviquat^{®} Hold (BASF SE)), Vinylpyrrolidon/Methacrylamid/Vinylimidazol/1-Vinyl-3-methyl-1H-imidazoliummethylsulfat-Copolymer (wie beispielsweise das mit der INCI-Bezeichnung Polyquaternium-68 unter der Handelsbezeichnung Luviquat^{®} Supreme (BASF SE)), sowie Gemische aus diesen Polymeren.

Weitere in den erfindungsgemäßen Mitteln bevorzugt einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Zu diesen Polymeren gehören beispielsweise Chitosane. Chitosan und/oder Chitosanderivate gelten als ganz besonders bevorzugt geeignete filmbildende und/oder festigende Polymere im Sinne der vorliegenden Erfindung. Chitosane stellen Biopolymere dar und werden zur Gruppe der Hydrokolloide gezählt. Chemisch betrachtet, handelt es sich um partiell deacetylierte Chitine unterschiedlichen Molekulargewichtes. Vorzugsweise werden solche Typen eingesetzt, die ein durchschnittliches Molekulargewicht von 800.000 bis 1.200.000 Dalton, eine Viskosität nach Brook-field (1 Gew.-%ig in Glycolsäure) unterhalb von 5000 mPas, einen Deacetylierungsgrad im Bereich von 80 bis 88 % und einem Aschegehalt von weniger als 0,3 Gew.-% aufweisen. Neben den Chitosanen als typischen kationischen Biopolymeren kommen im Sinne der Erfindung auch kationisch derivatisierte Chitosane (wie z. B. Quaternierungsprodukte) oder alkoxylierte Chitosane in Frage.

Erfindungsgemäß bevorzugte Mittel sind dadurch gekennzeichnet, daß sie als Chitosanderivat(e) Neutralisationsprodukte von Chitosan mit mindestens einer Säure, ausgewählt aus Milchsäure, Pyrrolidoncarbonsäure, Nicotinsäure, Hydroxyisobuttersäure, Hydroxyisovaleriansäure enthaltend oder Gemische dieser Neutralisationsprodukte umfassen. Geeignete Chitosan(derivate) sind beispielsweise unter den Handelsbezeichnungen Hydagen^{®} CMF (1 Gew.-% Aktivsubstanz in wässriger Lösung mit 0.4 Gew.-% Glykolsäure, Molekulargewicht 500000 bis 5000000 g/mol Cognis), Hydagen^{®} HCMF (Chitosan (zu 80 % deacetyliert), Molekulargewicht 50000 bis 1000000 g/mol, Cognis), Kytamer^{®} PC (80 Gew.-% Aktivsubstanz an Chitosan pyrolidoncarboxylat (INCI-Bezeichnung: Chitosan PCA), Amerchol) und Chitolam^{®} NB/101 im Handel frei verfügbar. Das Chitosan bzw. dessen Derivate sind in den erfindungsgemäßen Mitteln bevorzugt in einer Menge von 0,01 Gew.-% bis 20 Gew.-%, besonders bevorzugt von 0,01 Gew.-% bis 10,0 Gew.-%, ganz besonders bevorzugt von 0,1 Gew.-% bis 1 Gew.-%, jeweils bezogen auf das Gewicht des erfindungsgemäßen Mittels, enthalten.

Als im Sinne der Erfindung bevorzugt geeignete temporär kationische Polymere gelten gleichfalls solche, die mindestens eine Struktureinheit der Formeln (M1-1) bis (M1-8) aufweisen 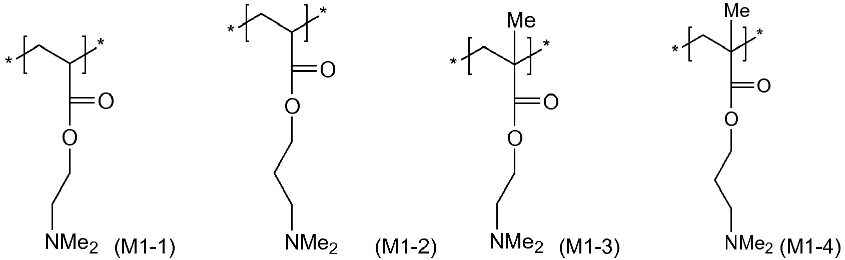 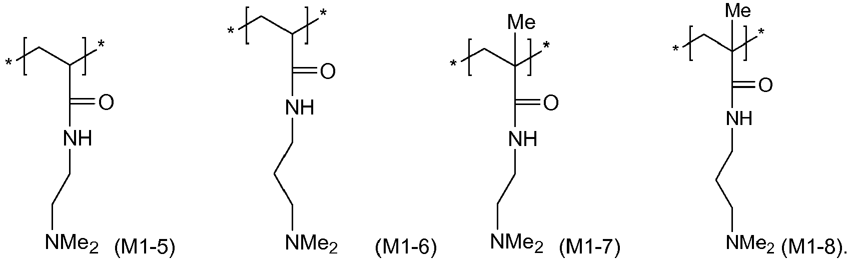 Dabei sind wiederum solche Copolymere bevorzugt, die mindestens eine Struktureinheit der Formeln (M1-1) bis (M1-8) und zusätzlich mindestens eine Struktureineheit der Formel (M10) enthalten, 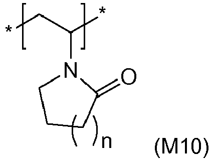 worin n 1 oder 3 bedeutet.

Dabei gilt wiederum die Gruppe der Polymere
- Vinylcaprolactam/Vinylpyrrolidon/Dimethylaminoethylmethacrylat-Copolymer (beispielsweise INCI-Bezeichnung: Vinyl Caprolactam/PVP/Di-methylaminoethyl Methacrylate Copolymer unter dem Handelsnamen Gaffix^{®} VC 713 (ISP)),
- Vinylpyrrolidon/Vinylcaprolactam/Dimethylaminopropylmethacrylamid-Copolymer (beispielsweise INCI-Bezeichnung: VP/Vinyl Caprolactam/DMAPA Acrylates Copolymer unter dem Handelsnamen Aquaflex^{®} SF-40 (ISP)),
- Vinylcaprolactam/Vinylpyrrolidon/Dimethylaminoethylmethacrylat-Copolymer (beispielsweise als.35-39% Festkörper in Ethanol in form des Handelsprodukts Advantage LC E mit der INCI-Bezeichnung: Vinyl Caprolactam/VP/Dimethylaminoethyl Methacrylate Copolymer, Alcohol, Lauryl Pyrrolidone (ISP)),
- Vinylpyrrolidon/Dimethylaminopropylmethacrylamid-Copolymer (beispielsweise INCI-Bezeichnung: VP/DMAPA Acrylates Copolymer unter dem Handelsnamen Styleze CC-10 (ISP)),
als bevorzugte Liste zur Auswahl.

Die erfindungsgemäßen Mittel können als filmbildendes und/oder festigendes Polymer auch mindestens ein amphoteres Polymer enthalten. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten.
Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Alkylestern darstellt.
Letztere weisen zusätzlich zu der kationogenen Gruppe bzw. positiv geladenen Gruppe mindestens eine negativ geladene Gruppe im Molekül auf und werden auch als zwitterionische Polymere bezeichnet. Die amphoteren Polymere sind in den erfindungsgemäßen Mitteln bevorzugt in Mengen von 0,01 Gew.-% bis 20 Gew.-%, besonders bevorzugt von 0,05 bis 10 Gew.-%, bezogen auf das gesamte Mittel, enthalten. Mengen von 0,1 bis 5 Gew.-% sind ganz besonders bevorzugt.

Weiterhin können als filmbildende und/oder festigende Polymere mindestens ein anionisches filmbildendes und/oder anionisches festigendes Polymer eingesetzt werden.
Bei den anionischen Polymeren handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.
Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.
Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.
Weitere bevorzugt einsetzbare anionische Polymere werden ausgewählt aus der Gruppe, die gebildet wird, aus
- Copolymeren aus Vinylacetat und Crotonsäure (wie sie beispielsweise als Handelsprodukt Aristoflex^{®} A 60 mit der INCI-Bezeichnung VA/Crotonates Copolymer von der Firma CIBA in einer 60 Gew.-%-igen Dispersion in Isopropanol-Wasser vermarktet werden),
- Copolymeren aus Ethylacrylat und Methacrylsäure (wie sie beispielsweise unter dem Handelsnamen Luviflex^{®} Soft mit einer Säurezahl von 84 bis 105 unter der INCI-Bezeichnung Acrylates Copolymer in einer ca. 20 bis 30 Gew.-%igen Dispersion in Wasser von der Firma BASF SE vertrieben werden),
- Polyurethanen mit mindestens einer Carboxylgruppe (wie beispielsweise ein Copolymer aus Isophthalsäure, Adipinsäure, 1,6-Hexandiol, Neopentylglykol und Isophorondiisocyanat wie es unter dem Handelsnamen Luviset PUR mit der INCI-Bezeichung Polyurethane-1 von der Firma BASF SE vertrieben wird).

Falls insbesondere stark verdickend wirkende anionische Polymere zum Einsatz kommen, sollte wiederum im Rahmen einer bevorzugten Ausführungsform darauf geachtet werden, dass das zuvor genannte bevorzugte Viskositätskriterium der erfindungsgemäßen Mittel eingehalten wird. Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Die erfindungsgemäßen Mittel enthalten bevorzugt zusätzlich mindestens einen von dem Carbonsäureester der Formel (I) verschiedenen Ölkörper.
Zu den natürlichen und synthetischen kosmetischen Ölkörpern sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe
- Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethylhexyl)-cyclohexan (Cetiol^{®} S) und Di-n-octylether (Cetiol^{®}OE) können bevorzugt sein.
- Esteröle. Unter Esterölen sind zu verstehen die Ester von C₆ - C₃₀ - Fettsäuren mit C₂ - C₃₀-Fettalkoholen. Bevorzugt sind die Monoester der Fettsäuren mit Alkoholen mit 2 bis 24 C-Atomen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat
- (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin.
Die Ölkörper sind wiederum bevorzugt in einer Menge von 5,0 bis 20 Gew.-%, insbesondere von 10 bis 20 Gew.-%, enthalten

Besonders bevorzugt enthält das erfindungsgemäße Mittel als Ölkörper mindestens ein flüssiges Paraffinöl. Dies ist wiederum bevorzugt in einer Menge von 5,0 bis 20 Gew.-%, insbesondere von 10 bis 20 Gew.-%, enthalten.

Zur Intensivierung des erfindungsgemäßen Effektes enthalten die erfindungsgemäßen Mittel zusätzlich mindestens ein anionisches in einer Menge von 1,0 bis 15,0 Gew.-% wobei das anionische Tensid ein Alkenyletherphosphat der Formel (T1), 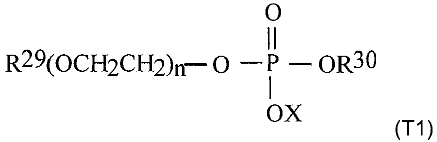 in der R²⁹ bevorzugt für einen ungesättigten Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄-Kohlenwasserstoffrest, steht, ist.

Zusätzlich können die erfindungsgemäßen Mittel weitere(s) Tensid(e) enthalten, wobei sich prinzipiell nichtionische, anionische, kationische, ampholytische Tenside eignen. Die zusätzlichen Tenside sind in dem erfindungsgemäß Mittel bevorzugt in einer Menge von 0,01 Gew.-% bis 30 Gew.-%, besonders bevorzugt von 5 Gew.-% bis 25 Gew.-%, jeweils bezogen auf das Gewicht des Mittels, enthalten.

Es hat sich als besonders bevorzugt erwiesen, wenn die erfindungsgemäßen Mittel zusätzlich mindestens ein nichtionisches Tensid enthalten.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 100 Mol Ethylenoxid und/oder 1 bis 5 Mol Propylenoxid an gesättigte oder ungesättigte, lineare oder verzweigte Fettalkohole mit 8 bis 30 C-Atomen, an Fettsäuren mit 8 bis 30 C-Atomen,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht.
Als ganz besonders bevorzugte nichtionische Tenside haben sich die Alkylenoxid-Anlagerungsprodukte an ungesättigte lineare Fettalkohole mit jeweils 2 bis 20 Mol Ethylenoxid pro Mol Fettalkohol bzw. Fettsäure erwiesen. Die nichtionischen Tenside sind erfindungsgemäß bevorzugt in einer Menge von 1,0 bis 20,0, insbesondere von 3,0 bis 15 Gew.-% in dem Mittel enthalten.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- Alkyl- und/oder Alkenyletherphosphate der Formel (T1), 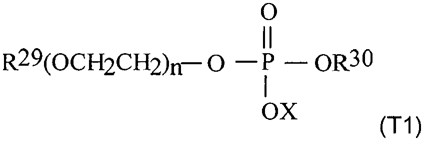 in der R²⁹ bevorzugt für einen aliphatischen Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄ - Kohlenwasserstoffrest, steht,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Die anionischen Tenside sind wiederum bevorzugt in einer Menge von 1,0 bis 15,0 Gew.-%, insbesondere von 3,0 bis 10,0 Gew.-%, enthalten.

Die erfindungsgemäßen Mittel enthalten mindestens ein anionisches Tenside in einer Menge von 1,0 bis 15,0 Gew.-%, wobei das anionische Tensid ein Alkenyletherphosphat der Formel (T1) ist, 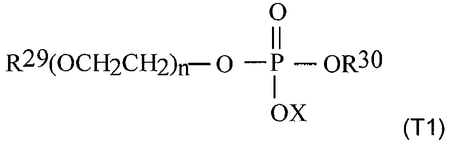 in der R²⁹ bevorzugt für einen ungesättigten Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄-Kohlenwasserstoffrest, steht. Ganz besonders bevorzugt werden die anionischen Tenside ausgewählt aus

PEG-3 Oleyletherphosphat (INCI-Bezeichnung: Oleth-3 Phosphate) und/oder PEG-5 Oleyletherphosphat (INCI-Bezeichnung: Oleth-5 Phosphate) und/oder PEG-10 Oleyletherphospat (INCI-Bezeichnung: Oleth-10 Phosphate).

Folgende Mittel sind erfindungsgemäß besonders bevorzugt:
(HH):
   Mittel zur Behandlung keratinhaltiger Fasern, insbesondere menschlichem Haar, enthaltend in Mengen, jeweils bezogen auf das Gewicht des gesamten Mittels, von
      (a) 0,1 bis 1,0 Gew.-% mindestens eines Carbonsäureesters der Formel (I) 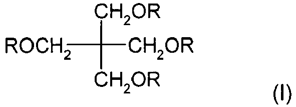 worin R unabhängig voneinander für eine verzweigte (C₈ bis C₃₀)-Acylgruppe oder ein Wasserstoffatom steht und höchstens einer der Reste R für ein Wasserstoffatom steht,
      (b) 2,0 bis 10 Gew.-% mindestens eines (C₂ bis C₆)-Diols,
      (c) 5,0 bis 20,0 Gew.-% mindestens eines Polyols,
      (d) Wasser,
      (e) 10 bis 20 Gew.-% mindestens eines Ölkörpers, insbesondere Paraffinöl,
      (f) 3,0 bis 15,0 Gew.-% mindestens eines nichtionischen Tensids,
      (g) 1,0 bis 10,0 Gew.-% mindestens eines anionischen Tensids ausgewählt unter den Alkenyletherphosphaten der Formel (T1), 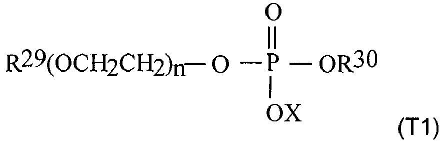 in der R²⁹ bevorzugt für einen ungesättigten Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄-Kohlenwasserstoffrest, steht.
         voneinander stehend für einen C₁ bis C₄-Kohlenwasserstoffrest, steht.
(JJ):
   Mittel zur Behandlung keratinhaltiger Fasern, insbesondere menschlichem Haar, enthaltend in Mengen, jeweils bezogen auf das Gewicht des gesamten Mittels, von
      (a) 0,1 bis 1,0 Gew.-% mindestens eines Carbonsäureesters der Formel (I) 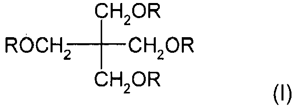 worin R unabhängig voneinander für eine verzweigte (C₈ bis C₃₀)-Acylgruppe steht,
      (b) 2,0 bis 10 Gew.-% mindestens eines (C₂ bis C₆)-Diols,
      (c) 5,0 bis 20,0 Gew.-% mindestens eines Polyols,
      (d) Wasser,
      (e) 10 bis 20 Gew.-% mindestens eines Ölkörpers, insbesondere Paraffinöl,
      (f) 3,0 bis 15,0 Gew.-% mindestens eines nichtionischen Tensids,
      (g) 1,0 bis 10,0 Gew.-% mindestens eines anionischen Tensids ausgewählt unter den Alkenyletherphosphaten der Formel (T1), 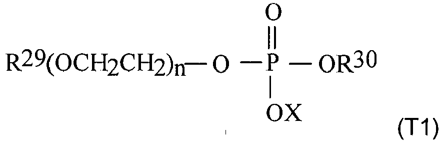 in der R²⁹ bevorzugt für einen ungesättigten Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄-Kohlenwasserstoffrest, steht.
   (LL): Mittel zur Behandlung keratinhaltiger Fasern, insbesondere menschlichem Haar, enthaltend in Mengen, jeweils bezogen auf das Gewicht des gesamten Mittels, von
      (a) 0,1 bis 1,0 Gew.-% Pentaerythritol tetraisostearat,
      (b) 2,0 bis 10 Gew.-% mindestens eines (C₂ bis C₆)-Diols,
      (c) 5,0 bis 20,0 Gew.-% mindestens eines Polyols,
      (d) Wasser,
      (e) 10 bis 20 Gew.-% mindestens eines Ölkörpers, insbesondere Paraffinöl,
      (f) 3,0 bis 15,0 Gew.-% mindestens eines nichtionischen Tensids,
      (g) 1,0 bis 10,0 Gew.-% mindestens eines anionischen Tensids ausgewählt unter den Alkenyletherphosphaten der Formel (T1), 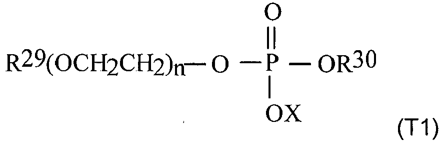 in der R²⁹ bevorzugt für einen ungesättigten Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄-Kohlenwasserstoffrest, steht.
   (NN):
      Mittel zur Behandlung keratinhaltiger Fasern, insbesondere menschlichem Haar, enthaltend in Mengen, jeweils bezogen auf das Gewicht des gesamten Mittels, von
         (a) 0,1 bis 1,0 Gew.-% Pentaerythritol tetraisostearat,
         (b) 2,0 bis 10,0 Gew.-% mindestens eines (C₂ bis C₆)-Diols, ausgewählt unter 1,2-Propylenglykol, Butan-1,3-diol, Butan-2,3-diol, 2-Methyl-2,4-butandiol, 2-Methyl-2,4-pentandiol, 1,5-Pentandiol, 2-Ethyl-1,3-hexandiol,
         (c) 5,0 bis 20,0 Gew.-% mindestens eines Polyols,
         (d) Wasser,
         (e) 10 bis 20 Gew.-% mindestens eines Ölkörpers, insbesondere Paraffinöl,
         (f) 3,0 bis 15,0 Gew.-% mindestens eines nichtionischen Tensids,
         (g) 1,0 bis 10,0 Gew.-% mindestens eines anionischen Tensids ausgewählt unter den Alkenyletherphosphaten der Formel (T1), 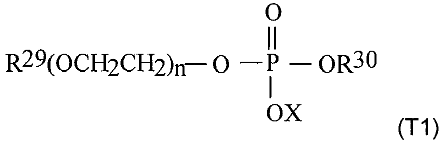 in der R²⁹ bevorzugt für einen ungesättigten Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄-Kohlenwasserstoffrest, steht.

Es ist gemäß Ausführungsformen (HH), (JJ), (LL) und (NN) wiederum bevorzugt, wenn als Diol eine Mischung aus 1,2-Propylenglykol und Butan-1,3-diol enthalten ist.

Das bevorzugte Polyol der Ausführungsformen (HH), (JJ), (LL) und (NN) wird ausgewählt unter den bevorzugten Polyolen (vide supra), insbesondere ist das Polyol Sorbitol.

Die Mittel weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Die erfindungsgemäßen Mittel können weiterhin die Hilfs- und Zusatzstoffe enthalten, die üblicherweise herkömmlichen Stylingmitteln zugesetzt werden. Als geeignete Hilfs- und Zusatzstoffe sind insbesondere zusätzliche Pflegestoffe zu nennen.

Als Pflegestoff kann das Mittel optional mindestens ein Proteinhydrolysat und/oder eines seiner Derivate enthalten. Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff kann das erfindungsgemäße Mittel weiterhin mindestens ein Vitamin, ein Provitamin, eine Vitaminvorstufe und/oder eines derer Derivate enthalten. Bevorzugt enthalten die erfindungsgemäßen Mittel Vitamine, Provitamine und Vitaminvorstufen aus den Gruppen A, B, C, E und H. Panthenol, Pantolacton, Pyridoxin und seine Derivate sowie Nicotinsäureamid und Biotin sind besonders bevorzugt. Ganz besonders bevorzugt wird als Pflegestoff D-Panthenol, gegebenenfalls in Kombination mit mindestens einem der oben genannten Silikonderivate eingesetzt.

Wird also ein besonders flexibler Halt gewünscht, können die erfindungsgemäßen Mittel statt oder zusätzlich zu Glycerin und/oder Propylenglykol Panthenol enthalten. In einer bevorzugten Ausführungsform enthalten die erfindungsgemäßen Mittel Panthenol, vorzugsweise in einer Menge von 0,05 bis 10 Gew.-%, besonders bevorzugt 0,1 - 5 Gew.-%, jeweils bezogen auf das gesamte Mittel.

Als Pflegestoff können die erfindungsgemäßen Mittel weiterhin mindestens einen Pflanzenextrakt enthalten. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Das Mittel kann weiterhin mindestens ein Lipid als Pflegestoff enthalten. Erfindungsgemäß geeignete Lipide sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV^{®} vertrieben. Die erfindungsgemäßen Mittel enthalten die Lipide bevorzugt in Mengen von 0,01 bis 10 Gew.-%, insbesondere 0,1 bis 5 Gew.-%, bezogen auf die gesamte Anwendungszubereitung. Durch Zugabe eines UV-Filters können sowohl die Mittel selbst, als auch die behandelten Fasern vor schädlichen Einflüssen von UV-Strahlung geschützt werden. Vorzugsweise wird daher dem Mittel mindestens ein UV-Filter zugegeben. Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.
In einer besonderen Ausführungsform enthält das erfindungsgemäße Mittel weiterhin einen oder mehrere direktziehende Farbstoffe. Direktziehende Farbstoffe sind üblicherweise Nitrophenylendiamine, Nitroaminophenole, Azofarbstoffe, Anthrachinone, Indophenole oder kationische direktziehende Farbstoffe eingesetzt. Weiterhin können die erfindungsgemäßen Mittel auch in der Natur vorkommende Farbstoffe enthalten, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind. Die erfindungsgemäßen Mittel gemäß dieser Ausführungsform enthalten die direktziehenden Farbstoffe bevorzugt in einer Menge von 0,001 bis 20 Gew.-%, bezogen auf das gesamte Mittel.
Es ist erfindungsgemäß bevorzugt, dass die erfindungsgemäßen Mittel frei von Oxidationsfarbstoffvorprodukten sind. Oxidationsfarbstoffvorprodukte werden eingeteilt in sogenannte Entwicklerkomponenten und Kupplerkomponenten.

Die Formulierung der erfindungsgemäßen Mittel kann in allen für Stylingmittel üblichen Formen erfolgen, beispielsweise in Form von Cremes, Emulsionen, Wachsen, Gelen oder auch tensidhaltigen schäumenden Lösungen oder anderen Zubereitungen, die für die Anwendung auf dem Haar geeignet sind. Vorzugsweise werden die erfindungsgemäßen Mittel als Haarcreme oder Haargel, insbesondere als Haargel, konfektioniert.
Ein zweiter Gegenstand der Erfindung ist die Verwendung der erfindungsgemäßen Mittel zur temporären Verformung von Haaren und/oder zur Haarpflege.
Die erfindungsgemäßen Mittel und Produkte, die diese Mittel enthalten, insbesondere Haargele oder Haarcremes, zeichnen sich insbesondere dadurch aus, dass sie dem behandelten Haar einen guten Frisurenhalt verleihen und das Haar einen natürlichen Glanz erhält.
Es ist erfindungsgemäß bevorzugt, das Mittel des ersten Erfindungsgegenstandes als leave-on Haarbehandlungsmittel zu verwenden.

Ein dritter Gegenstand der Erfindung ist ein Verfahren zur Behandlung keratinhaltiger Fasern, insbesondere menschlicher Haare, worin das erfindungsgemäße Mittel des ersten Erfindungsgegenstandes auf die keratinhaltigen Fasern appliziert wird.
Es ist erfindungsgemäß bevorzugt, wenn die keratinhaltigen Fasern vor, während oder nach der Applikation des erfindungsgemäßen Mittels in Form gebracht werden.
Ferner gilt es erfindungsgemäß als bevorzugt, im Rahmen des erfindungsgemäßen Verfahrens das erfindungsgemäße Mittel nicht aus den keratinhaltigen Fasern auszuspülen.

## Patentansprüche

1. Mittel zur Behandlung keratinhaltiger Fasern, insbesondere menschlichem Haar, enthaltend in Mengen, jeweils bezogen auf das Gewicht des gesamten Mittels, von
(a) 0,1 bis 1,0 Gew.-% mindestens eines Carbonsäureesters der Formel (I) 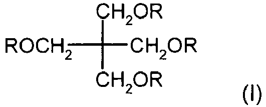 worin R unabhängig voneinander für eine verzweigte (C₈ bis C₃₀)-Acylgruppe oder ein Wasserstoffatom steht und höchstens einer der Reste R für ein Wasserstoffatom steht,
(b) 2,0 bis 10,0 Gew.-% mindestens eines (C₂ bis C₆)-Diols,
(c) 5,0 bis 20,0 Gew.-% mindestens eines Polyols,
(d) Wasser,
wobei zusätzlich mindestens ein anionisches Tensid in einer Menge von 1,0 bis 15,0 Gew.-% enthalten ist, wobei das anionische Tensid ein Alkenyletherphosphat der Formel (T1), 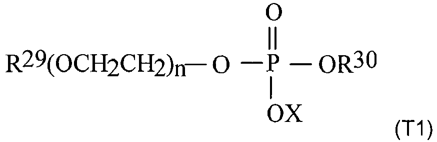 in der R²⁹ bevorzugt für einen ungesättigten Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄-Kohlenwasserstoffrest, steht, ist.

2. Mittel nach Anspruch 1, **dadurch gekennzeichnet, dass** gemäß Formel (I) alle Reste R unabhängig voneinander für eine verzweigte (C₈ bis C₃₀)-Acylgruppe stehen.

3. Mittel nach einem der Ansprüche 1 oder 2, **dadurch gekennzeichnet, dass** R unabhängig voneinander steht für 2-Ethylhexanoyl, 2-Octyldodecanoyl, Isostearoyl, Isocetanoyl, Cetostearoyl, Hexyllauroyl oder Isononanoyl, insbesondere für Isostearoyl.

4. Mittel nach einem der Ansprüche 1 bis 3, **dadurch gekennzeichnet, dass** die (C₂ bis C₆)-Diole ausgewählt werden aus mindestens einem Diol der Gruppe, die gebildet wird aus 1,2-Propylenglykol, Butan-1,3-diol, Butan-2,3-diol, 2-Methyl-2,4-butandiol, 2-Methyl-2,4-pentandiol, 1,5-Pentandiol.

5. Mittel nach einem der Ansprüche 1 bis 4, **dadurch gekennzeichnet, dass** die Polyole eine chemische Struktur aufweisen, die ausschließlich aus Kohlenstoffatomen, Wasserstoffatomen und Sauerstoffatomen aufgebaut ist und ein Kohlenwasserstoffgerüst aufweist, an das mindestens drei Hydroxygruppen (insbesondere vier Hydroxygruppen) binden und an das die Sauerstoffatome lediglich als Hydroxygruppen (-OH) und/oder als Etherfunktionalitäten (-O-) an das Kohlenwasserstoffgerüst binden.

6. Mittel nach einem der Ansprüche 1 bis 5, **dadurch gekennzeichnet, dass** das Polyol ausgewählt wird aus mindestens einer Verbindung der Gruppe, die gebildet wird aus Glyzerin, 1,2,4-Pentantriol, 1,3,4-Pentantriol, 1,3,5-Pentantriol, Sorbitol, Inositol, Xylitol, Mannitol, Gluconolacton, Glucuronsäure, 1,2,6-Hexantriol, Hydroxyethylsorbitol, Phytantriol, Hydroxypropylphytantriol, Lactitol, Maltitol, Pentaerythritol, Polyglycerin-3, Glucose, Lactose, Maltose, Polyglycerin-4, Polyglycerin-6, Polyglycerin-10, Polyglycerin-20, Polyglycerin-40, Tripropylenglykol, insbesondere Glyzerin und/oder Sorbitol.

7. Mittel nach einem der Ansprüche 1 bis 6, **dadurch gekennzeichnet, dass** zusätzlich mindestens ein Ölkörper enthalten ist.

8. Mittel nach einem der Ansprüche 1 bis 7, **dadurch gekennzeichnet, dass** zusätzlich mindestens ein nichtionisches Tensid enthalten ist.

## Claims

1. An agent for treating keratin-containing fibers, in particular human hair, containing, in amounts of, in each case based on the weight of the total agent:
(a) from 0.1 to 1.0 wt.% of at least one carboxylic acid ester of formula (I) 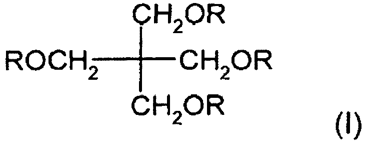 in which R represents, independently of one another, a branched (C₈ to C₃₀) acyl group or a hydrogen atom, and at most one of the groups R represents a hydrogen atom,
(b) from 2.0 to 10.0 wt.% of at least one (C₂ to C₆) diol,
(c) from 5.0 to 20.0 wt.% of at least one polyol,
(d) water, wherein at least one anionic surfactant is additionally contained in an amount of from 1.0 to 15.0 wt.%, wherein the anionic surfactant is an alkenyl ether phosphate of formula (T1), 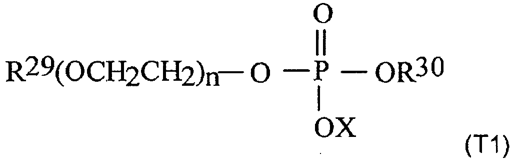 in which R²⁹ preferably represents an unsaturated hydrocarbon group having 8 to 30 carbon atoms, R³⁰ represents hydrogen, a (CH₂CH₂O)ₙR²⁹ group or X, n represents numbers from 1 to 10, and X represents hydrogen, an alkali metal or alkaline-earth metal, or NR³¹R³²R³³R³⁴, R³¹ to R³⁴ representing, independently of one another, a C₁ to C₄ hydrocarbon group.

2. The agent according to claim 1, **characterized in that** all the groups R represent, independently of one another, a branched (C₈ to C₃₀) acyl group in accordance with formula (I).

3. The agent according to claim 1 or 2, **characterized in that** R represents, independently of one another, 2-ethylhexanoyl, 2-octyldodecanoyl, isostearoyl, isocetanoyl, cetostearoyl, hexyllauroyl or isononanoyl, in particular isostearoyl.

4. The agent according to one of claims 1 to 3, **characterized in that** the (C₂ to C₆) diols are selected from at least one diol of the group that is formed by 1,2-propylene glycol, butane-1,3-diol, butane-2,3-diol, 2-methyl-2,4-butanediol, 2-methyl-2,4-pentanediol, 1,5-pentanediol.

5. The agent according to one of claims 1 to 4, **characterized in that** the polyols have a chemical structure that is composed exclusively of carbon atoms, hydrogen atoms and oxygen atoms and comprises a hydrocarbon backbone to which at least three hydroxyl groups (in particular four hydroxyl groups) bond, and to which the oxygen atoms only bond to the hydrocarbon backbone as hydroxyl groups (-OH) and/or as ether functionalities (-O-).

6. The agent according to one of claims 1 to 5, **characterized in that** the polyol is selected from at least one compound of the group formed by glycerol, 1,2,4-pentanetriol, 1,3,4-pentanetriol, 1,3,5-pentanetriol, sorbitol, inositol, xylitol, mannitol, gluconolactone, glucuronic acid, 1,2,6-hexanetriol, hydroxyethyl sorbitol, phytantriol, hydroxypropyl phytantriol, lactitol, maltitol, pentaerythritol, polyglycerol-3, glucose, lactose, maltose, polyglycerol-4, polyglycerol-6, polyglycerol-10, polyglycerol-20, polyglycerol-40, tripropylene glycol, in particular glycerol and/or sorbitol.

7. The agent according to one of claims 1 to 6, **characterized in that** at least one oil component is additionally contained.

8. The agent according to one of claims 1 to 7, **characterized in that** at least one non-ionic surfactant is additionally contained.

## Revendications

1. Agent de traitement de fibres kératiniques, en particulier de cheveu humain, présent dans des quantités, par rapport au poids de l'ensemble de l'agent respectivement, de
(a) 0,1 à 1,0 % en poids d'au moins un ester d'acide carboxylique de formule (I) 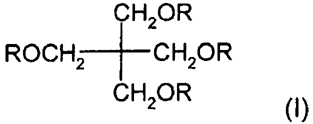 où R représente, sans interdépendance, un groupe acyle ramifié en (C₈ à C₃₀) ou un atome d'hydrogène et où au maximum un des radicaux R représente un atome d'hydrogène,
(b) 2,0 à 10,0 % en poids d'au moins un diol en (C₂ à C₆),
(c) 5,0 à 20,0 % en poids d'au moins un polyol,
(d) eau,
au moins un tensioactif anionique étant présent en plus dans une quantité de 1,0 à 15,0 % en poids, le tensioactif anionique étant un alcényléther-phophate de formule (T1) 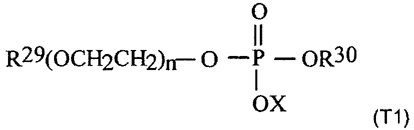 où R²⁹ représente de préférence un radical hydrocarboné non saturé comportant 8 à 30 atomes de carbone, R³⁰, l'hydrogène, un radical (CH₂CH₂O)ₙR²⁹ ou X, n, des nombres de 1 à 10 et X, l'hydrogène, un métal alcalin ou alcalino-terreux, ou NR³¹R³²R³³R³⁴, R³¹ à R³⁴ représentant indépendamment les uns des autres un radical hydrocarboné en C₁ à C₄.

2. Agent selon la revendication 1 **caractérisé en ce que**, selon la formule I, tous les radicaux R représentent indépendamment les uns des autres un groupe acyle ramifié en (C₈ à C₃₀).

3. Agent selon une des revendications 1 ou 2 **caractérisé en ce que** R représente, sans interdépendance, le 2-éthylhexanoyle, 2-octyldodécanoyle, isostéaroyle, isocétanoyle, cétostéaroyle, hexyllauroyle, ou isononanoyle, en particulier l'isostéaroyle.

4. Agent selon l'une quelconque des revendications 1 à 3 **caractérisé en ce que** les diols (en C₂ à C₆) sont choisis à partir d'au moins un diol du groupe constitué du 1,2-propylène glycol, butane-1,3-diol, butane-2,3-diol, 2-méthyle-2,4-butanediol, 2-méthyle-2,4-pentanediol, 1,5-pentanediol.

5. Agent selon l'une quelconque des revendications 1 à 4 **caractérisé en ce que** les polyols présentent une structure chimique constituée exclusivement d'atomes de carbone, d'atomes d'hydrogène et d'atomes d'oxygène et présentant un squelette d'hydrocarbure auquel sont liés au moins trois groupes hydroxy (en particulier quatre groupes hydroxy) et auquel les atomes d'oxygène sont liés uniquement en tant que groupes hydroxy (-OH) et/ou en tant que fonctions éther (-O-).

6. Agent selon l'une quelconque des revendications 1 à 5 **caractérisé en ce que** le polyol est choisi parmi au moins un composé du groupe constitué de la glycérine, du 1,2,4-pentanetriol, du 1,3,4-pentanetriol, du 1,3,5-pentanetriol, du sorbitol, de l'inositol, du xylitol, du mannitol, de la gluconolactone, de l'acide glucuronique, du 1,2,6-hexanetriol, de l'hydroxyéthyl sorbitol, du phytantriol, de l'hydroxypropylphytantriol, du lactitol, du maltitol, du pentaérythritol, de la polyglycérine-3, du glucose, du lactose, du maltose, de la polyglycérine-4, de la polyglycérine-6, de la polyglycérine-10, de la polyglycérine-20, de la polyglycérine-40, du tripropylène glycol, en particulier de la glycérine et/ou du sorbitol.

7. Agent selon l'une quelconque des revendications 1 à 6 **caractérisé en ce qu'**en plus, au moins un corps huileux est présent.

8. Agent selon l'une quelconque des revendications 1 à 7 **caractérisé en ce qu'**en plus, au moins un tensioactif non ionique est présent.
